(19) Europäisches Patentamt / European Patent Office / Office européen des brevets

(11) **EP 1 575 558 B1**

(12) **FASCICULE DE BREVET EUROPEEN**

(45) Date de publication et mention de la délivrance du brevet:
**08.11.2017  Bulletin 2017/45**

(51) Int Cl.:
*A61K 9/00* *(2006.01)*          *A61K 45/06* *(2006.01)*
*A61K 47/02* *(2006.01)*          *A61K 47/18* *(2017.01)*
*A61K 31/401* *(2006.01)*          *A61K 31/728* *(2006.01)*

(21) Numéro de dépôt: **03814501.7**

(22) Date de dépôt: **26.12.2003**

(86) Numéro de dépôt international:
**PCT/FR2003/003917**

(87) Numéro de publication internationale:
**WO 2004/060348 (22.07.2004 Gazette 2004/30)**

(54) **UTILISATION OPHTALMIQUE ET OPHTALMOLOGIQUE D'UNE BASE NUTRITIVE COMPLEXE EN MILIEU AQUEUX**

OPHTALMISCHE UND OPHTALMOLISCHE VERWENDUNG EINER KOMPLEXEN ERNÄHRUNGSGRUNDLAGE IN WÄSSRIGEN MEDIEN

OPHTHALMIC AND OPHTHALMOLOGICAL USE OF A COMPLEX NUTRITIVE BASE IN AN AQUEOUS MEDIUM

(84) Etats contractants désignés:
**AT BE BG CH CY CZ DE DK EE ES FI FR GB GR HU IE IT LI LU MC NL PT RO SE SI SK TR**

(30) Priorité:  **26.12.2002  FR 0216722**

(43) Date de publication de la demande:
**21.09.2005  Bulletin 2005/38**

(73) Titulaire: **Thorel, Jean-Noël**
**75014 Paris (FR)**

(72) Inventeurs:
• **THOREL, Jean-Noël**
  **F-75014 Paris (FR)**
• **GATTO, Hugues**
  **F-06570 Saint-Paul (FR)**

(74) Mandataire: **Cabinet Laurent & Charras**
**Le Contemporain**
**50 Chemin de la Bruyère**
**69574 Dardilly Cedex (FR)**

(56) Documents cités:
EP-A- 0 468 122          EP-A- 0 517 970
EP-A- 0 698 388          WO-A-94/15649
WO-A-96/21421          WO-A-02/060495

WO-A-02/087326          FR-A- 2 425 858
US-A- 5 654 266          US-A- 5 942 218
US-A1- 2002 049 281          US-B1- 6 194 457

• DATABASE WPI Section Ch, Week 199018 Derwent Publications Ltd., London, GB; Class A96, AN 1990-135663 XP002252714 & JP 02 083318 A (ZERIA SHINYAKU KOGYO KK) 23 mars 1990 (1990-03-23)
• DATABASE WPI Section Ch, Week 200239 Derwent Publications Ltd., London, GB; Class B05, AN 2002-355876 XP002252715 & JP 2002 020279 A (TAISHO PHARM CO LTD) 23 janvier 2002 (2002-01-23)
• BERMAN M B: "COLLAGENASE INHIBITORS: RATIONALE FOR THEIR USE IN TREATING CORNEAL ULCERATION" INTERNATIONAL OPHTHALMOGY CLINICS, LITTLE, BROWN AND CO., BOSTON, US, vol. 15, no. 4, janvier 1975 (1975-01), pages 49-66, XP008011256 ISSN: 0020-8167 cité dans la demande
• BELLON G ET AL: "EFFECTS OF PREFORMED PROLINE AND PROLINE AMINO ACID PRECURSORS INCLUDING GLUTAMINE ON COLLAGEN SYNTHESIS IN HUMAN FIBROBLAST CULTURES" BIOCHIMICA ET BIOPHYSICA ACTA, vol. 930, no. 1, 1987, pages 39-47, XP002290392 ISSN: 0006-3002 cité dans la demande

Il est rappelé que: Dans un délai de neuf mois à compter de la publication de la mention de la délivrance du brevet européen au Bulletin européen des brevets, toute personne peut faire opposition à ce brevet auprès de l'Office européen des brevets, conformément au règlement d'exécution. L'opposition n'est réputée formée qu'après le paiement de la taxe d'opposition. (Art. 99(1) Convention sur le brevet européen).

EP 1 575 558 B1

• VARMA S D: "SCIENTIFIC BASIS FOR MEDICAL THERAPY OF CATARACTS BY ANTIOXIDANTS" AMERICAN JOURNAL OF CLINICAL NUTRITION, BETHESDA,MD, US, vol. 53, no. 1, SUPPL, 1991, pages 335S-345S, XP009003151 ISSN: 0002-9165

**Description**

La présente invention concerne l'utilisation ophtalmique et ophtalmologique d'une base nutritive complexe, aussi bien chez l'homme que chez l'animal.

Par "ophtalmique" on entend qu'une base nutritive complexe telle que définie et décrite ci-après peut être mise en oeuvre dans diverses

applications, non thérapeutiques, en relation avec l'oeil chez l'homme ou l'animal, et plus précisément en relation avec la surface extérieure ou l'extérieur de la cornée.

Par "ophtalmologique" on entend que la même base nutritive complexe peut être mise en oeuvre dans le traitement thérapeutique ou clinique de l'oeil chez l'homme ou l'animal, et plus précisément en application, par exemple locale, au contact extérieur de la cornée.

Plus particulièrement, aux fins des utilisations précédemment définies, la présente invention s'intéresse aux compositions trophiques comprenant une base nutritive complexe.

Par base nutritive complexe, on entend toute composition ou

formulation, en milieu aqueux, se distinguant, comme précisé ci-après, d'un milieu de culture cellulaire, même si elle permet, en général, comme ce dernier, une culture in vitro viable pendant au moins 72 heures d'un inoculum de certaines cellules prédéterminées.

Différents milieux de cultures ont été effectivement décrits, et sont

commercialisés. Ainsi, s'agissant de la culture in vitro de kératinocytes, on peut citer :

- BOYCE ST, HAM RG, Calcium-regulated differentiation of normal human epidermal keratinocytes in defined clonai culture and serum-free serial culture ; J. Invest. Dermatol. 1983; 81: 335-409 ;
- BOYCE ST, HAM RG, Cultivation, frozen storage, and clonai growth of normal human epidermal keratinocytes in serum-free media ; J. Tissue Culture Methods 1985 ; 9 : 83-93 ;
- le milieu commercial dénommé MCDB 153, commercialisé notamment par les Sociétés IRVINE SCIENTIFIC et GIBCO-BRL;
- les milieux commerciaux dénommés DMEM (DUBECO Modified Epidermal Medium); KSFM de GIBCO-BRL, etc...

De tels milieux de cultures incorporent, pour être actifs, des facteurs de croissance cellulaire, soit que ces facteurs sont compris au départ dans la composition du milieu de culture, soit que ces facteurs sont produits au moment de la culture, par exemple par une couche nourricière de fibroblastes, s'agissant de la culture de kératinocytes.

Une base nutritive complexe, telle que considérée selon la présente invention, ne comporte pas dans sa mise en oeuvre de facteur de croissance, par exemple, d'EGF (Epidermal Growth Factor).

Beaucoup des milieux de culture précédemment identifiés comportent des extraits biologiques, par exemple d'origine animale, cellulaire, ou autre, c'est-à-dire obtenus à partir d'une matière première biologique. Au rang de ces extraits biologiques, on peut citer, à titre d'exemple, tout sérum de veau foetal, tout extrait de tige pituitaire de boeuf.

Par nature, ces extraits ont une composition variable, voire indéterminée.

Une base nutritive complexe, telle que considérée selon la présente invention, ne comporte aucun extrait biologique tel que défini précédemment.

Ces mêmes milieux de culture précédemment identifiés comprennent, dans certains cas, différents principes actifs thérapeutiques, utilisés en tant que médicaments, et ce pour favoriser la conservation, et/ou, l'efficacité du milieu de culture.

Au rang de ces principes actifs, on peut citer certains antibiotiques, par exemple la pénicilline et/ou la streptomycine, seules ou en mélange, certaines hormones, par exemple la toxine cholérique, l'insuline.

Une base nutritive complexe, considérée selon la présente invention, ne comporte pas de principe actif médicamenteux.

De telles bases nutritives complexes, au sens où celles-ci peuvent être utilisées, seules ou en combinaison avec d'autres composants, en tant que produit actif ou comme excipient, ont été décrites dans le document WO 96/21421.

De telles bases nutritives complexes sont constituées en milieu aqueux par au moins, une multiplicité d'acides aminés, dont certains essentiels, différentes vitamines, des oligo-éléments, et des sels métalliques.

Conformément au document WO 96/21421, une telle base nutritive complexe a, par exemple, la composition suivante, selon Tableau 1 ci-après :

**Tableau 1**

| COMPOSANTS | Concentration en mg/l |
|------------|----------------------:|
| Eau | q.s.p. |

EP 1 575 558 B1

(suite)

| COMPOSANTS | Concentration en mg/l |
|---|---|
| **Acides aminés** | |
| L-Alanine | 9,2 |
| L-Arqinine HCL | 421,4 |
| **Acides aminés (suite)** | |
| L-Asparagine (anhydre) | 14,2 |
| Acide L-aspartique | 4,0 |
| L-Cystéine HCl.$H_2O$ | 42,0 |
| Acide L-glutamique | 14,8 |
| L-Glutamine | 1754,4 |
| Glycine | 7,6 |
| L-Histidine HCl.$H_2O$ | 50,0 |
| L-Isoleucine | 6,0 |
| L-Leucine | 131,2 |
| L-Lysine HCl | 54,0 |
| L-Méthionine | 13,5 |
| L-Phénylalanine | 10,0 |
| L-Proline | 34,6 |
| L-Sérine | 126,1 |
| L-Thréonine | 24,0 |
| L-Tryptophane | 9,3 |
| L-Tyrosine 2 Na 2$H_2O$ | 11,7 |
| L-Valine | 70,3 |
| | |
| **Vitamines** | |
| d-Biotine | 0,02 |
| Acide folique | 0,80 |
| Nicotinamide | 0,04 |
| D-Ca Pantothénate | 0,30 |
| Pyridoxine HCl | 0,06 |
| Riboflavine | 0,04 |
| Thiamine HCl | 0,30 |
| i-Inositol | 18,0 |
| Pyruvate de sodium | 55,0 |
| Thymidine | 0,73 |
| Adénine (HCl) | 24,0 |
| Acide DL-lipoïque | 0,20 |
| | |
| **Composants inorganiques** | |
| Chlorure de sodium | 6800,0 |

4

(suite)

| COMPOSANTS | Concentration en mg/l |
|---|---|
| KCl | 112,0 |
| $Na_2HPO_4$ | 284,0 |
| $CuSO_4.5H_2O$ | 0,003 |
| Acétate de sodium | 300,0 (anhydre) |
| D-Glucose | 1080,0 |
| HEPES (pipérazine) | 6600,0 |
| Phosphoryléthanolamine | 0,06768 |
| Ethanolamine | 0,04684 |
| Sulfate de sodium | 3,4 |
| Bicarbonate de sodium | 1160,0 |
| $FeSO_4.7H_2O$ | 1,39 |
| $MgCl_2.6H_2O$ | 120,0 |
| $CaCl_2.2H_2O$ | De 13,0 à 22,05 |
| $ZnSO_4.7H_2O$ | 0,144 |
| $(NH_4)_6Mo_7O_{24}.4H_2O$ | 0,00120 |
| $Na_2SiO_3.5H_2O$ | 0,142 |
| **Composants inorganiques** (suite) | |
| $MnCl_2.4H_2O$ | 0,00002 |
| $SnCl_2.2H_2O$ | 0,00011 |
| $NH_4VO_3$ | 0,00057 |

Conformément au document WO 02 087326, on décrit et propose une composition ophtalmique ou ophtalmologique comprenant un dérivé vitaminique, en tant qu'agent tensio-actif, non ionique, et un agent ou conservateur cationique, par exemple le polyhexaméthylène biguanide, et un agent désinfectant, par exemple du peroxyde d'hydrogène. Cette composition a essentiellement des propriétés nettoyantes et désinfectantes, mais non régénérantes au niveau de la cornée.

Conformément au document US 5 654 266, on décrit et propose une composition essentiellement saline et tamponnée pour la conservation et le rinçage de tissus avant une transplantation, par exemple des cornées destinées à être greffées. La composition proposée comprend à titre principal du βhydroxy-butyrate, diminuant la production et l'accumulation d'acide lactique dans les tissus stockés.

Conformément au document EP 0 517 970, on décrit et propose une solution pour l'irrigation intra-oculaire des chambres antérieure et postérieure de l'oeil, à des fins de chirurgie oculaire, avec des propriétés réparatrices uniquement de l'endothélium cornéen, c'est-à-dire dans la chambre antérieure de l'oeil.

Conformément au document JP 02 083318, on décrit une composition nettoyante de l'oeil comprenant des sels d'ammonium quaternaire, du chondroitine-sulfate de sodium et de l'acide glycyrrhétinique, ainsi que des alcohols, dont propylène-glycol.

Conformément au document WO 026495, on décrit une composition d'entretien d'une lentille de contact, en particulier pour son imprégnation, comprenant des vitamines, l'objectif poursuivi étant de relarguer ladite composition au contact de la cornée, à partir de la lentille de contact imprégnée.

Le document US 6 194 457 B1, décrit et propose une solution pour traiter les irritations, la sécheresse de l'oeil et/ou traiter la cataracte, comprenant des acides aminés, des vitamines, des oligo-éléments, des sels métalliques, de la cystéine et exclut notamment tout facteur de croissance cellulaire ou composé permettant une cicatrisation.

La présente invention a pour objet l'utilisation ophtalmique ou ophtalmologique d'une base nutritive complexe telle que précédemment définie, par exemple par l'intermédiaire d'une composition trophique comprenant ladite base, en particulier pour améliorer la viabilité, par exemple en cas d'agression ou de stress, et maintenir l'intégrité et l'équilibre des cellules épithéliales de la cornée de l'oeil, chez l'homme ou l'animal.

Plus particulièrement, la présente invention a pour objet une telle utilisation pour améliorer la viabilité, la croissance, et la différenciation des cellules de l'épithélium cornéen, chez l'homme ou l'animal.

Conformément à la présente invention, la composition trophique en milieu aqueux pouvant être mise en oeuvre comprend :

- une base nutritive complexe, constituée, au moins, par une multiplicité d'acides aminés, des vitamines, des oligo-éléments, et des sels métalliques, ladite base excluant tout facteur de croissance cellulaire, tout extrait biologique d'origine animale ou cellulaire, et tout principe actif thérapeutique, la pénicilline, la streptomycine, la toxine cholérique et l'insuline pour obtenir un médicament ophtalmologique ou une solution ophtalmologique, formulé pour une application au contact extérieur de l'oeil chez l'homme ou l'animal, où le médicament ophtalmologique ou la solution ophtalmologique consiste en une composition trophique en milieu aqueux comprenant la base nutritive complexe, un inhibiteur des collagénases de l'épithélium cornéen chez l'homme ou l'animal choisi dans le groupe constitué par la cystéine, la N-acétyl cystéine, et le sel de calcium de l'EDTA, et un promoteur de la synthèse du néocollagène qui est la proline ou l'hydroxyproline.

Préférentiellement la composition trophique est formulée, avec la base nutritive complexe, pour établir un pH compris entre 7,3 et 7,5, et une osmolarité comprise entre 300 et 350 mOsm.

Différents inhibiteurs de collagénase sont déjà connus, fonctionnellement et/ou chimiquement, et on se référera enparticulieur aux documents suivants :

- Berman; Int. Ophtalmol. Clin. 1975 Winter ; 15(4) : 49-66 - Haffner JC, Fecteau KA, Eilet H, Vet. Ophtalmol. 2003 Mar; 6(1): 67-72.

Différents promoteurs de la synthèse du néocollagène sont déjà connus, et on se référera en particulier aux documents suivants :

- Bellon G, Monboisse JC, Fandoux A, Borel JP, Biochim. Biophys. Acta. 1987, Aug 19, 930 (1) : 39-47
- Kefalides NA, Cameron JD, Tomicheck EA, Yanoff M, J Biol Chem. 1976, Feb 10 ; 251(3) : 730-3.

Selon l'invention, la composition trophique présente avantageusement les caractéristiques suivantes, ces dernières étant considérées seules ou en combinaison:

- l'inhibiteur des collagénases est la N-acétyl cystéine ;
- l'inhibiteur des collagénases représente au maximum 5 %, et de préférence entre 0,05 et 0,5 % en poids de ladite composition ;
- le promoteur de la synthèse du néocollagène représente au maximum 0,5 %, et de préférence 0,004 % en poids de ladite composition ;
- la composition trophique comprend de l'acide hyaluronique, et/ou un sel de l'acide hyaluronique, dans une proportion pondérale totale de la composition, au plus égale à 0,1 %, et de préférence égale à 0,07 % ;
- la composition trophique comprend un conservateur, dans une proportion pondérale de la composition, au plus égale à 0,0001 % ;
- le conservateur est le polyhexanide ou polyhexaméthylène biguanide (PHMB) ;
- la composition trophique répond à la formule décrite par le Tableau 2 dans la description.

Par rapport à la composition selon le tableau 1, la composition selon le Tableau 2 diffère de cette dernière sur les points suivants :

- elle ne comprend pas de vitamine B12, de putrescine 2HCL, de $Na_2SiO_3$
- elle comprend, et un inhibiteur de collagènase, et un promoteur de la synthèse du néocollagène
- ses caractéristiques de pH et d'osmolarité permettent 35 l'oxygénation de l'épithélium cornéen et l'activité du lysozyme des larmes.

La présente demande décrit également l'utilisation d'une composition trophique telle que précédemment décrite en tant que médicament en ophtalmologie ou contactologie, chez l'homme ou l'animal, par application, par exemple locale, au contact extérieur de la cornée.

L'invention concerne également un tel médicament, sous forme liquide, ou sous forme sèche, c'est-à-dire pour reconstitution avec un milieu aqueux.

Dans une variante, un tel médicament est sous forme liquide, par exemple sous la forme de gouttes, ou de larmes régénérantes, ou de collyre, ou de solution.

Ainsi, à titre d'exemple, la présente invention propose un collyre comprenant une composition trophique telle que définie précédemment, permettant de cicatriser la cornée, ou pour prévenir les adhérences conjonctivales.

Un tel collyre a les indications suivantes :

- ulcérations cornéennes d'origine traumatique ou autre, brûlures de la cornée ;
- prévention des adhérences conjonctivales et cornéo-conjonctivales (symblépharon) ;
- xerosis conjonctival et cornéen.

L'invention concerne également une solution ophtalmologique, par exemple, de confort, pour application locale au contact extérieur de la cornée, chez l'homme ou chez l'animal, comprenant par exemple une composition trophique telle que précédemment définie.

Ainsi, la présente invention propose, par exemple, « des larmes régénérantes », comprenant une composition trophique telle que définie précédemment, pour l'hydratation de lentilles de contact et la régénération de la cornée, chez l'homme ou l'animal.

De telles « larmes régénérantes » lubrifient accessoirement la cornée. En définitive, elles évitent ou limitent l'érosion prématurée de la cornée, sous l'action de lentilles de contact, rigides ou semi-rigides en particulier. Et ces « larmes régénérantes » améliorent le confort visuel, et augmentent la durée de port possible de lentilles de contact.

Ainsi, la présente invention propose, par exemple, des gouttes de confort, pour les utilisations suivantes :

- sécheresses oculaires d'origine iatrogène (liées notamment à l'emploi de rétinoïdes topiques ou systémiques, d'antihistaminiques,

d'antiparkinsoniens, de betabloquants, de spasmolytiques, d'anxiolytiques, de neuroleptiques, d'antidépresseurs et de bronchodilatateurs, etc...), et entraînant un inconfort, en particulier chez les patients porteurs de lentilles de contact ;

- sécheresses oculaires chez les personnes âgées.

La présente demande décrit également l'utilisation d'une base nutritive complexe telle que précédemment définie pour le traitement, par exemple la conservation, de pièces ou prothèses, par exemple lentilles de contact, agencées pour venir au contact par l'extérieur de la cornée oculaire, chez l'homme ou l'animal.

Elle concerne à ce titre une solution pour la conservation, ou le stockage, ou le transport ou la mise en oeuvre (par exemple chirurgicale)de pièces ou prothèses, par exemple lentilles de contact, agencées pour venir au contact par l'extérieur de la cornée oculaire, chez l'homme ou l'animal. Grâce à une base nutritive complexe telle que précédemment définie, et en particulier à une composition trophique selon la présente invention, et comme démontré par les études exposées ci-après, les propriétés intrinsèques primaires de l'épithélium cornéen sont préservées durablement, tout en augmentant sa résistance aux agressions, et favorisant, le cas échéant, son retour à un état d'équilibre.

La présente invention est maintenant décrite et exemplifiée, à partir d'une composition trophique en milieu aqueux, ayant la formule selon Tableau 2.

## Tableau 2

| NBRE LIGNE | DENOMINATION INTERNATIONALE NOMENCLATURE COSMETIQUE INGREDIENT (NOM INCI) | CONCENTRATION En mg/l | NBRE LIGNE | DENOMINATION INTERNATIONALE NOMENCLATURE COSMETIQUE INGREDIENT (NOM INCI) | CONCENTRATION En mg/l |
|---|---|---|---|---|---|
| 1 | EAU | q.s.p. | 14 | SODIUM PYRUVATE | 55 |
| 2 | CHLORURE DE SODIUM | 6800 | 15 | LYSINE HCL | 54 |
| | | | 16 | HISTIDINE HCL | 50 |
| 3 | GLUTAMINE | 1754,4 | 17 | CYSTEINE HCL | 42 |
| 4 | SODIUM BICARBONATE | 1160 | 18 | ADENINE | 24 |
| 5 | GLUCOSE | 1080 | 19 | THREONINE | 24 |
| 6 | ARGININE HCL | 421,4 | 20 | CHLORURE DE Ca | 20,05 |
| 7 | SODIUM ACETATE | 300 | 21 | INOSITOL | 18 |
| 8 | DISODIUM PHOSPHATE | 284 | 22 | ACIDE GLUTAMIQUE | 14,8 |
| 9 | LEUCINE | 131,2 | 23 | ASPARAGINE | 14,2 |
| 10 | SERINE | 126,1 | 24 | METHIONINE | 13,5 |
| 11 | CHLORURE DE Mg | 120,0 | 25 | TYROSINE | 11,7 |
| 12 | CHLORURE DE K | 112 | 26 | PHENYLALANINE | 10,0 |
| 13 | VALINE | 70,3 | 27 | TRYPTOPHANE | 9,3 |

| NBRE LIGNE | DENOMINATION INTERNATIONALE NOMENCLATURE COSMETIQUE INGREDIENT (NOM INCI) | CONCENTRATION En mg/l |
|---|---|---|
| 28 | ALANINE | 9,2 |
| 29 | GLYCINE | 7,6 |
| 30 | ISOLEUCINE | 6,0 |
| 31 | ACIDE ASPARTIQUE | 4,0 |
| 32 | SODIUM SULFATE | 3,4 |
| 33 | SULFATE FERREUX | 0,003 |
| 34 | ACIDE FOLIQUE | 0,8 |
| 35 | THYMIDINE | 0,73 |
| 36 | CYANOCOBALAMINE | 0,41 |
| 37 | CALCIUM PANTOTHENATE | 0,3 |
| 38 | THIAMINE HCL | 0,3 |
| 39 | ACIDE THIOCTIQUE | 0,3 |
| 40 | ZINC SULFATE | 0,144 |
| 41 | SODIUM SILICATE | 0,142 |
| 42 | PYRODIXINE HCL | 0,06 |
| 43 | NIACINAMIDE | 0,04 |
| 44 | RIBOFLAVIN | 0,3 |
| 45 | BIOTIN | 0,02 |
| 46 | SULFATE DE CUIVRE | 0,003 |
| 47 | AMMONIUM MOLYBDATE | 0,00120 |
| 48 | AMMONIUM VANADATE | 0,003 |
| 49 | CHLORURE DE Mn | 0,00002 |
| 50 | HYALURONATE DE SODIUM | 70 |
| 51 | POLYHEXANIDE ou POLYHEXAMETHYLENE BIGUANIDE | 0,1 |
| 52 | N-ACETYL-CYSTEINE | 500 |
| 53 | HYDROXYPROLINE ou PROLINE | 35 |

Conformément à la définition selon la présente invention de la composition trophique, les ingrédients en milieu aqueux de la base nutritive complexe sont tous ceux numérotés de 1 à 49, tandis que les ingrédients 50 à 53 sont considérés comme extérieurs à la base nutritive complexe, et permettent son adaptation à un contact externe avec l'épithélium de la cornée de l'oeil.

Les ingrédients 50 et 51 sont optionnels, en fonction du rôle ou de la fonction recherchée pour la composition trophique.

Par ailleurs, le choix et les proportions des différents ingrédients sont définis pour établir dans la composition trophique finalement obtenue :

- un pH compris entre 7,3 et 7,5, et préférentiellement compris entre 7,42 et 7,45 (pH favorisant l'oxygénation de l'épithélium cornéen et l'activité du lyzosyme des larmes).
- une osmolarité comprise entre 300 et 350 mOsm, et par exemple, égale à 340 mOsm.

### Etude n°1

On évalue la biocompatibilité de la composition trophique précédemment définie sur un modèle d'épithélium de cornée humain reconstitué.

A partir de la formule selon Tableau 2, on définit et formule quatre variantes, appelées respectivement :

EVE 1 : sans Hyaluronate de sodium (ingrédient 50) et sans conservateur (ingrédient 51).
EVE 2 : sans Hyaluronate de sodium (ingrédient 50) et avec conservateur (ingrédient 51).
EVE 3 : avec Hyaluronate de sodium (ingrédient 50) et sans conservateur (ingrédient 51).
EVE 4 : identique à la formule selon Tableau 2, et donc avec Hyaluronate de sodium (ingrédient 50), et avec conservateur (ingrédient 51).

Des ajustements minimes des autres ingrédients sont apportés, pour maintenir le pH et l'osmolarité dans les valeurs précédemment énoncées.

La cytocompatibilité des formules EVE 1 à EVE 4 est évaluée par application topique sur un modèle in vitro d'épithélium de cornée humain reconstruit, tel que disponible auprès de la Société SKINETHIC LABORATORIES, 45 Rue Saint Philippe, 06000 NICE, France.

Roger W. BEUERMAN et Lia PEDROSA ont décrit, par le détail, l'ultra-structure de la cornée humaine, dans un article intitulé « Ultra-structure of the human cornea », dans le journal « Microscopy Research and Technique », 33 : 320-335 (1996), et on s'y référera en tant que de besoin.

A partir de la structure naturelle, O. DOUCET et al. ont proposé et décrit un épithélium de cornée humaine, tridimensionnel, reconstruit, en particulier pour effectuer différents essais in vitro de toxicité ; cf. l'article ayant pour titre « A new in vitro human epithelium model for assessing the eye irritation potential of formulated cosmetic products », paru dans « In vitro and Molecular Toxicology », 11, 4 : 273-283 (1998).

Cet épithélium de cornée reconstruit, ou modèle, est obtenu par culture de cellules épithéliales de cornée, par exemple la lignée HCE, disponible et commercialisée par LSU Eye Center, Louisiana State University School of Medicine, New Orleans, Louisiana, 70112 USA. Les cellules épithéliales sont cultivées à l'interface air/liquide dans un milieu défini, et elles forment un tissu épithélial cornéen, dépourvu de stratum corneum, s'approchant de l'épithélium cornéen in vivo.

L'étude est réalisée sur de tels épithéliums cornéens reconstruits, tels qu'obtenus au 5ème jour de culture (taille : 0,5 cm$^2$), le contrôle qualité des différents épithéliums aux différents stade de culture étant assuré par la Société SKINETHIC LABORATORIES, précitée, conformément à la publication dernière citée.

Le principe du test consiste à appliquer la composition trophique directement au contact de l'épithélium cornéen reconstruit, 15 minutes trois fois par jour sur une période de 7 jours. On réalise en parallèle un témoin épithélium cornéen non traité. Chaque condition (à l'exception du témoin non traité) est réalisée en double. Aux 2ème, 4ème et 7ème jours d'expérimentation, la viabilité cellulaire (test au MTT) est évaluée pour chacune des conditions.

En pratique, 30 µl des compositions trophiques à tester sont déposés 3 fois par jour (à 9 h, 13 h et à 17 h), directement à la surface des équivalents cornéens, pendant 15 minutes. Après cette période de contact, les compositions en excès sont éliminées et les épithéliums maintenus en incubation à 37°C sous atmosphère humide, 5 % $CO_2$. Cette opération est répétée tous les jours pendant 7 jours. Aux 2ème, 4ème et 7ème jours d'expérimentation, les épithéliums reconstruits sont prélevés pour effectuer le test de viabilité cellulaire.

Aux 2ème, 4ème et 7ème jours d'expérimentation, les épithéliums prélevés sont rincés au PBS et placés dans 300 µl de MTT (0,5 mg/ml). Après 3 heures d'incubation à 37°C, 5 % $CO_2$, les épithéliums sont transférés dans 1,5 ml d'isopropanol. L'extraction du produit de clivage du MTT est réalisée sous légère agitation, pendant 2 heures à température ambiante. La densité optique à 570 nm est ensuite mesurée sur 200 µl de solution, d'extraction. Les résultats sont exprimés en pourcentage de viabilité par rapport au témoin (non traité) :

$$\% \text{ viabilité} = [DO_{(570\text{ nm produit testé})} / DO_{(570\text{ nm témoin})}] \times 100$$

Dans les conditions expérimentales ainsi définies, on observe un maintien total de la viabilité in vitro des épithéliums de cornée humain reconstruits, après application topique de chacune des compositions trophiques précitées. La présence du conservateur et de l'acide hyaluronique aux concentrations d'utilisation ainsi définies n'influe pas sur la viabilité cellulaire des épithéliums qui est maintenue.

### Etude n°2

Par mise en culture d'un épithélium de cornée humain reconstitué, dans les compositions trophiques précédemment définies, on compare les résultats obtenus par rapport à ceux obtenus par mise en culture dans une solution saline tamponnée.

Le modèle d'épithélium cornéen retenu est à l'identique de celui décrit et défini dans l'Etude n°1, mis en culture pendant 72 heures dans la composition trophique étudiée.

Une seule composition trophique est retenue pour cette étude, à savoir celle définie par le Tableau 2, appelée EVE 4 dans l'Etude n°1.

Dès réception des épithéliums, le milieu nutritif de transport SKINETHIC est éliminé et remplacé, pour un des épithéliums, par 1 ml de la composition trophique étudiée. Un second épithélium est placé, pour comparaison, dans une solution saline tamponnée (Phosphate Buffered Saline, PBS, enrichi en $Ca^{2+}$ et $Mg^{2+}$). La cytocompatibilité de la composition trophique étudiée a été préalablement vérifiée (absence d'effet cytotoxique, cf. Etude précédente n°1). Les épithéliums sont maintenus en incubation à 37°C sous atmosphère humide, 5 % $CO_2$. Les milieux sont renouvelés tous les jours. Après 3 jours de culture, les 2 épithéliums reconstruits sont prélevés pour effectuer une analyse histologique. Ils sont fixés par une solution de formaldéhyde à 10 % puis inclus dans la paraffine. L'histologie des coupes colorées à l'hematoxyline/éosine est évaluée en microscopie optique.

L'interprétation histopathologique des coupes prend en compte l'épaisseur et la régularité du tissu épithélial ainsi que la morphologie, la cohésion et la capacité des cellules à proliférer.

Concernant l'épithélium cultivé pendant 72 h dans la composition trophique, l'analyse du prélèvement montre un épithélium implanté sur le support membranaire. Il comporte 6 à 7 couches de cellules jointives (les ponts d'union sont visibles). Il n'y a pas de signe de nécrose ni de kératinisation de la couche superficielle.

Dans le cas de l'épithélium cultivé pendant 72 h dans une solution saline tamponnée, l'analyse du prélèvement montre une disparition quasi-totale de l'épithélium, seul le support est visible. Il n'y a pas de couche cellulaire identifiable.

Dans les conditions expérimentales définies pour cette étude, on observe la survie et la cohésion cellulaire d'un épithélium de cornée humain reconstruit, mis en culture pendant 72 h dans une composition trophique selon la présente invention. En comparaison, l'expérimentation similaire réalisée avec une solution saline tamponnée montre une dégénérescence complète de l'épithélium, qui n'est plus identifiable.

### Etude n°3

On évalue les propriétés cicatrisantes de compositions trophiques selon l'invention, sur un modèle de cornée humain reconstitué.

Les quatre variantes de la composition trophique étudiée sont identiques à celles définies et décrites dans l'Etude n°1.

Le modèle d'épithélium cornéen, reconstruit, est à l'identique de celui décrit et défini dans les Etudes n°1 et n°2.

Les propriétés cicatrisantes des compositions trophiques étudiées sont évaluées après application topique sur le modèle précité, sur lequel est réalisée au préalable une lésion par altération mécanique.

La réalisation des lésions à la surface des épithéliums cornéens reconstruits est opérée à l'aide de la pointe d'un scalpel partageant sur leur diamètre chacun des épithéliums.

Le principe du test consiste à appliquer, immédiatement après le traumatisme, la composition trophique directement au contact de l'épithélium cornéen reconstruit, 15 minutes trois fois par jour, et ce quotidiennement sur une période de 9 jours. On réalise en parallèle un contrôle avec une solution saline tamponnée (PBS). Aux 2ème, 4ème, 7ème et 9ème jours d'expérimentation, l'histologie du tissu reconstruit est évaluée pour chacune des conditions. Elle est comparée à l'histologie d'une condition témoin de référence correspondant à un épithélium non agressé non traité.

En pratique, 30 $\mu$l des compositions trophiques à tester sont déposés 3 fois par jour (à 9 h, 13 h et à 17 h) directement à la surface des épithéliums cornéens pendant 15 minutes. Après cette période de contact, les compositions en excès sont éliminées et les épithéliums maintenus en incubation à 37°C sous atmosphère humide, 5 % $CO_2$. Cette opération est répétée tous les jours pendant 9 jours. Aux 2ème, 4ème, 7ème et 9ème jours d'expérimentation, les épithéliums reconstruits sont prélevés pour effectuer l'analyse histologique.

Aux 2$^{ème}$, 4$^{ème}$, 7$^{ème}$ et 9$^{ème}$ jours d'expérimentation, les épithéliums sont fixés par une solution de formaldéhyde à 10 %, puis inclus dans la paraffine. L'histologie des coupes colorées à l'hematoxyline/éosine est évaluée en microscopie optique.

L'évaluation des propriétés cicatrisantes des compositions trophiques est réalisée par analyse histologique des coupes et observation de la réparation de l'épithélium au niveau de la lésion induite par le scalpel. La localisation des lésions est effectuée par marquage à l'encre de Chine à la surface des épithéliums cornéens, avant d'effectuer la coupe histologique (coupe transversale de l'épithélium).

L'interprétation histopatholoqique des coupes prend en compte l'épaisseur et la régularité du tissu épithélial, ainsi que la morphologie, la cohésion et la capacité des cellules à proliférer après le traumatisme opéré.

Des photographies regroupées établissent, pour chaque condition, les coupes transversales des épithéliums réalisées en début de traitement (2$^{ème}$ jour après réalisation de la lésion) et au dernier jour de celui-ci (9$^{ème}$ jour).

Dans le cas du Témoin (non traité, non agressé), on observe, au 2$^{ème}$ jour de culture, un épithélium vivace composé de 6 à 7 couches de cellules, régulières dans leur forme (aspect pavimenteux), jointives. Au 9$^{ème}$ jour de traitement, on observe un épaississement de l'épithélium formé d'une dizaine de couches de cellules, toujours jointives et régulières. On note la présence d'une couche kératinisée en surface.

Dans le cas du traitement avec EVE 4 (composition trophique selon Tableau 2), on observe, sur la photographie prise au 2$^{ème}$ jour, la lésion réalisée au niveau de l'épithélium, mettant à nu le support membranaire. De part et d'autre de la plaie, l'épithélium est vivace. Il n'y a pas de kératinisation. Au 9$^{ème}$ jour de traitement, on observe au niveau du repère réalisé à l'encre de Chine, un épithélium réparé, continu, formé de plusieurs couches de cellules relativement régulières (d'aspect plus arrondi que dans le cas du Témoin), non nécrosées. On note la présence d'une zone de kératinisation dans les couches superficielles.

Dans le cas du traitement avec une solution saline tamponnée (PBS), on visualise au 2$^{ème}$ jour la zone lésée, marquée par une perte de substance importante au niveau de l'épithélium. Au 9$^{ème}$ jour, on observe au niveau du repère un épithélium réparé, constitué de 5 à 6 couches de cellules seulement, d'aspect arrondi, turgescentes pour certaines, avec la présence de vacuoles.

Dans le cas du traitement avec les compositions dites EVE 1, EVE 2, EVE 3, on observe, pour les prélèvements réalisés au 2$^{ème}$ jour d'expérimentation, la lésion repérée par une perte de substance totale. De part et d'autre, l'épithélium est pluristratifié, vivace et non kératinisé.

Au 9$^{ème}$ jour, pour la formulation EVE 1, on observe un épithélium reconstitué, continu, formé de cellules arrondies, assez régulières avec présence de quelques vacuoles. Pour les formulations EVE 2 et EVE 3, les épithéliums ont un aspect identique, avec des cellules jointives et de morphologie régulière.

Cependant, l'épaisseur des épithéliums apparaît plus mince par rapport à celle des épithéliums traités par la formulation EVE 4.

Dans les conditions expérimentales ainsi définies, on observe pour chacune des conditions testées une réparation des épithéliums de cornée humains préalablement lésés, au terme des traitements.

La formulation EVE 4 (contenant acide hyaluronique et conservateur) permet d'obtenir les meilleurs résultats. L'épithélium réparé est continu, vivace, pluristratifié, composé de cellules jointives et régulières.

**Etude n°4**

On évalue les propriétés cicatrisantes de compositions trophiques selon la présente invention, en comparaison avec une solution saline tamponnée.

La composition trophique étudiée est à l'identique de celle définie par le Tableau 2.

Le modèle d'épithélium cornéen, reconstruit, est à l'identique de celui mis en oeuvre dans les Etudes n$^{os}$ 1 à 3.

Les propriétés cicatrisantes des compositions trophiques sont évaluées par application topique sur un modèle in vitro d'épithélium de cornée humain reconstruit, sur lequel est réalisée, au préalable, une lésion par traumatisme mécanique.

La réalisation des lésions à la surface des épithéliums cornéens reconstruits est opérée à l'aide de la pointe d'un scalpel partageant sur leur diamètre chacun des épithéliums.

Le principe du test consiste à appliquer, immédiatement après le traumatisme, la composition trophique directement au contact de l'épithélium cornéen reconstruit, 15 minutes trois fois par jour, et ce quotidiennement sur une période de 7 jours. On réalise en parallèle un contrôle en solution saline tamponnée (PBS enrichi en Ca$^{2+}$ et Mg$^{2+}$). Aux 2$^{ème}$, 4$^{ème}$ et 7$^{ème}$ jours d'expérimentation, l'histologie du tissu reconstruit est évaluée pour chacune des conditions. Elle sera comparée à l'histologie d'une condition témoin de référence correspondant à un épithélium non agressé non traité.

En pratique, 30 $\mu$l des compositions à tester sont déposés 3 fois par jour (à 9 h, 13 h et à 17 h) directement à la surface des équivalents cornéens pendant 15 minutes. Après cette période de contact, les compositions en excès sont éliminées et les épithéliums maintenus en incubation à 37°C sous atmosphère humide, 5 % $CO_2$. Cette opération est répétée tous les jours pendant 7 jours. Aux 2ème, 4ème et 7ème jours d'expérimentation, les épithéliums reconstruits sont prélevés pour effectuer l'analyse histologique.

Aux 2ème, 4ème et 7ème jours d'expérimentation, les épithéliums sont fixés par une solution de formaldéhyde à 10 % puis inclus dans la paraffine. L'histologie des coupes colorées à l'hematoxyline/éosine est évaluée en microscopie optique.

L'évaluation des propriétés cicatrisantes des compositions trophiques est réalisée par analyse histologique des coupes et observation de la réparation de l'épithélium au niveau de la lésion induite par le scalpel. La localisation des lésions est effectuée par marquage à l'encre de Chine à la surface des épithéliums de cornée avant d'effectuer la coupe histologique (coupe transversale de l'épithélium).

L'interprétation histopathologique des coupes prend en compte l'épaisseur et la régularité du tissu épithélial ainsi que la morphologie, la cohésion et la capacité des cellules à proliférer après le traumatisme opéré.

Des photographies établissent, pour chaque condition, les coupes transversales des épithéliums réalisées aux différentes étapes du traitement.

Dans le cas du Témoin (non traité, non agressé), on observe, au 2ème jour de culture, un épithélium vivace composé de 6 à 7 couches de cellules, régulières dans leur forme (aspect pavimenteux), jointives. Au 7ème jour de traitement, on observe un épaississement de l'épithélium formé d'une dizaine de couches de cellules, toujours jointives et régulières. On note la présence d'une couche kératinisée en surface.

Dans le cas du traitement avec la composition trophique selon tableau 2, on observe, sur la photographie prise au 2ème jour, la lésion réalisée au niveau de l'épithélium mettant à nu le support membranaire. De part et d'autre de la plaie, l'épithélium est vivace. Il n'y a pas de kératinisation. Au 4ème jour, on observe une recolonisation cellulaire du support. L'épithélium comporte 3 à 4 couches de cellules dont certaines sont en division. Au 7ème jour de traitement, on observe au niveau du repère réalisé à l'encre de Chine, un épithélium réparé, continu, formé de plusieurs couches de cellules relativement régulières (d'aspect plus arrondi que dans le cas du Témoin), non nécrosées. On note la présence d'une zone de kératinisation dans les couches superficielles.

Dans le cas du traitement avec une solution saline tamponnée (PBS), on visualise au 2ème jour la zone lésée marquée par une perte de substance importante au niveau de l'épithélium. Au 4ème jour, l'épithélium se reconstitue, composé seulement d'une à deux couches de cellules arrondies. Au 7ème jour, l'épithélium est réparé, constitué de 5 à 6 couches de cellules, d'aspect arrondi, turgescentes pour certaines, avec la présence de vacuoles.

Dans les conditions expérimentales ainsi définies, la composition trophique permet la reconstitution d'un épithélium de cornée humain préalablement lésé. L'épithélium réparé est continu, vivace, pluristratifié, composé de cellules jointives et régulières.

L'application d'une solution saline tamponnée permet également une réparation de l'épithélium. Celui-ci n'est cependant formé que de 3 à 4 couches de cellules, témoin d'une régénération inférieure à celle observée avec la composition trophique. D'autre part, les cellules sont arrondies avec présence de nombres vacuoles.

## Revendications

1. Utilisation d'une base nutritive complexe en milieu aqueux, constituée au moins par une multiplicité d'acides aminés, des vitamines, des oligo-éléments, et des sels métalliques, ladite base excluant tout facteur de croissance cellulaire, ou tout extrait biologique d'origine animale ou cellulaire, la pénicilline, la streptomycine, la toxine cholérique et l'insuline, pour obtenir un médicament ophtalmologique, ou solution ophtalmologique, formulé pour une application au contact extérieur de l'oeil chez l'homme ou l'animal, où le médicament ophtalmologique ou la solution ophtalmologique consiste en une composition trophique en milieu aqueux comprenant la base nutritive complexe, un inhibiteur des collagénases de l'épithélium cornéen chez l'homme ou l'animal choisi dans le groupe constitué par la cystéine, la N-acétyl cystéine, et le sel de calcium de l'EDTA, et un promoteur de la synthèse du néocollagène qui est la proline ou l'hydroxyproline.

2. Utilisation selon la revendication 1, **caractérisée en ce que** la composition trophique est formulée pour établir un pH compris entre 7,3 et 7,5 et une osmolarité comprise entre 300 et 350 Osm.

3. Utilisation selon la revendication 1, **caractérisée en ce que** l'inhibiteur des collagénases est la N-acétyl cystéine.

4. Utilisation selon la revendication 1, **caractérisée en ce que** l'inhibiteur des collagénases représente au maximum 5 %, et de préférence entre 0,05 et 0,5 % en poids de la composition trophique.

5. Utilisation selon la revendication 1, **caractérisée en ce que** le promoteur de la synthèse du néocollagène représente au maximum 0,5 %, et de préférence 0,004 % en poids de la composition trophique.

**6.** Utilisation selon la revendication 1, **caractérisée en ce qu'**elle comprend de l'acide hyaluronique, et/ou un sel de l'acide hyaluronique, dans une proportion pondérale totale de la composition trophique, au plus égale à 0,1 %, et de préférence égale à 0,07 %.

**7.** Utilisation selon la revendication 1, **caractérisée en ce que** la composition tropique comprend un conservateur, dans une proportion pondérale de ladite composition, au plus égale à 0,0001 %.

**8.** Utilisation selon la revendication 7, **caractérisée en ce que** le conservateur est le polyhexanide ou polyhexaméthylène biguanide.

**9.** Utilisation selon la revendication 7, **caractérisée en ce que** in composition trophique répond à la formule décrite par le Tableau 2 dans la description.

**10.** Utilisation selon la revendication 1, **caractérisée en ce que** le médicament ophtalmologique ou la solution ophtalmologique est sous forme liquide, ou sous forme sèche, c'est-à-dire pour reconstitution avec un milieu aqueux.

**11.** Utilisation selon la revendication 1, **caractérisée en ce que** le médicament ophtalmologique ou la solution ophtalmologique est sous la forme de gouttes ou de larmes régénérantes, ou de gouttes de confort, ou de collyre, ou de solution.

**12.** Utilisation selon la revendication 1, **caractérisée en ce que** la solution ophtalmologique est une solution de confort.


**Patentansprüche**

**1.** Verwendung einer komplexen Nährstoffbasis in einem wässrigen Medium, die zumindest aus einer Vielzahl von Aminosäuren, Vitaminen, Spurenelementen und Metallsalzen gebildet ist, wobei die Basis jeglichen Zellwachstumsfaktor oder jeglichen biologischen Extrakt tierischen oder zellulären Ursprungs, Penicillin, Streptomycin, Choleratoxin und Insulin ausschließt, um ein ophthalmologisches Medikament oder eine ophthalmologische Lösung zu erhalten, die für eine Anwendung mit äußerlicher Berührung des Auges beim Menschen oder beim Tier formuliert ist, wobei das ophthalmologische Medikament oder die ophthalmologische Lösung aus einer trophischen Zusammensetzung in einem wässrigen Medium besteht, welche die komplexe Nährstoffbasis, einen Hemmer der Kollagenasen des Hornhautepithels beim Menschen oder beim Tier, ausgewählt aus der Gruppe, bestehend aus Cystein, N-Acetylcystein und dem Calciumsalz von EDTA, sowie einen Promotor der Neokollagensynthese, wobei es sich um Prolin oder Hydroxyprolin handelt, umfasst.

**2.** Verwendung gemäß Anspruch 1, **dadurch gekennzeichnet, dass** die trophische Zusammensetzung so formuliert ist, um einen pH-Wert im Bereich zwischen 7,3 und 7,5 und eine Osmolarität im Bereich zwischen 300 und 350 Osm festzulegen.

**3.** Verwendung gemäß Anspruch 1, **dadurch gekennzeichnet, dass** es sich bei dem Hemmer der Kollagenasen um N-Acetylcystein handelt.

**4.** Verwendung gemäß Anspruch 1, **dadurch gekennzeichnet, dass** der Hemmer der Kollagenasen höchstens 5 und vorzugsweise zwischen 0,05 und 0,5 Gew.-% der trophischen Zusammensetzung ausmacht.

**5.** Verwendung gemäß Anspruch 1, **dadurch gekennzeichnet, dass** der Promotor der Neokollagensynthese höchstens 0,5 und vorzugsweise 0,004 Gew.-% der trophischen Zusammensetzung ausmacht.

**6.** Verwendung gemäß Anspruch 1, **dadurch gekennzeichnet, dass** sie Hyaluronsäure und/oder ein Hyaluronsäuresalz in einem Gesamtgewichtsanteil, bezogen auf die trophische Zusammensetzung, von höchstens 0,1% und vorzugsweise 0,07% umfasst.

**7.** Verwendung gemäß Anspruch 1, **dadurch gekennzeichnet, dass** die trophische Zusammensetzung ein Konservierungsmittel in einem Gewichtsanteil, bezogen auf die Zusammensetzung, von höchstens 0,0001% umfasst.

**8.** Verwendung gemäß Anspruch 7, **dadurch gekennzeichnet, dass** es sich bei dem Konservierungsmittel um Polyhexanid oder Polyhexamethylenbiguanid handelt.

**9.** Verwendung gemäß Anspruch 7, **dadurch gekennzeichnet, dass** die trophische Zusammensetzung der in Tabelle 2 der Beschreibung beschriebenen Formel entspricht.

**10.** Verwendung gemäß Anspruch 1, **dadurch gekennzeichnet, dass** das ophtalmologische Medikament oder die ophtalmologische Lösung in flüssiger Form oder in trockener Form, d.h., für die Rekonstitution mit einem wässrigen Medium, vorliegt.

**11.** Verwendung gemäß Anspruch 1, **dadurch gekennzeichnet, dass** das ophtalmologische Medikament oder die ophtalmologische Lösung in Form von Tropfen oder regenerierender Tränenflüssigkeit oder Komfort-Tropfen oder Augentropfen oder einer Lösung vorliegt.

**12.** Verwendung gemäß Anspruch 1, **dadurch gekennzeichnet, dass** die ophtalmologische Lösung eine Komfort-Lösung ist.

**Claims**

**1.** Use of a complex nutritive base in an aqueous medium, consisting of at least a multiplicity of amino acids, vitamins, trace elements, and metallic salts, said base being free of any cellular growth factor, or any biological extract of animal or cellular origin, penicillin, streptomycin, cholera toxin and insulin, to obtain an ophthalmologic medicine, or an ophthalmologic solution, formulated for local application in external contact with human or animal eye, wherein the ophthalmologic medicine or the ophthalmologic solution consisting of a trophic composition in an aqueous medium comprising the complex nutritive base, an inhibitor of collagenases of the human or animal corneal epithelium selected from the group consisting of cysteine, N-acetylcysteine, and EDTA calcium salt, and a promotor of neocollagen synthesis which is proline or hydroxyproline.

**2.** Use according to Claim 1, **characterized in that** the trophic composition is formulated so as to establish a pH between 7.3 and 7.5 and an osmolarity between 300 and 350 Osm.

**3.** Use according to Claim 1, **characterized in that** the inhibitor of collagenases is N-acetylcysteine.

**4.** Use according to Claim 1, **characterized in that** the inhibitor of collagenases represents at most 5%, and preferably between 0.05 and 0.5% by weight of the trophic composition.

**5.** Use according to Claim 1, **characterized in that** the promotor of neocollagen synthesis represents at most 0.5%, and preferably 0.004% by weight of the trophic composition.

**6.** Use according to Claim 1, wherein it includes hyaluronic acid, and/or a salt of hyaluronic acid, in a total proportion by weight of the trophic composition of at most 0.1%, and preferably equal to 0.07%.

**7.** Use according to Claim 1, **characterized in that** the trophic composition comprises a preservative, in a proportion by weight of the composition of at most 0.0001%.

**8.** Use according to Claim 7, **characterized in that** the preservative is polyhexanide or polyhexamethylene biguanide.

**9.** Use according to Claim 7, **characterized in that** the trophic composition responds to the formula described in Table 2 of the specification.

**10.** Use according to Claim 1, **characterized in that** the ophthalmologic medicine or the ophthalmologic solution is in liquid form, or in dry form, which is for reconstitution with an aqueous medium.

**11.** Use according to Claim 1, **characterized in that** the ophthalmologic medicine or the ophthalmologic solution is in drops or regenerating tears form, or comfort drops, eyewash, or solution.

**12.** Use according to Claim 1, **characterized in that** the ophthalmologic solution is a comfort solution.

**RÉFÉRENCES CITÉES DANS LA DESCRIPTION**

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- WO 9621421 A
- WO 02087326 A
- US 5654266 A
- EP 0517970 A
- JP 2083318 A
- WO 026495 A
- US 6194457 B1

**Littérature non-brevet citée dans la description**

- **BOYCE ST ; HAM RG.** Calcium-regulated differentiation of normal human epidermal keratinocytes in defined clonai culture and serum-free serial culture. *J. Invest. Dermatol.,* 1983, vol. 81, 335-409
- **BOYCE ST ; HAM RG.** Cultivation, frozen storage, and clonai growth of normal human epidermal keratinocytes in serum-free media. *J. Tissue Culture Methods,* 1985, vol. 9, 83-93
- **BERMAN.** *Int. Ophtalmol. Clin.,* 1975, vol. 15 (4), 49-66
- **HAFFNER JC ; FECTEAU KA ; EILET H.** *Vet. Ophtalmol.,* Mars 2003, vol. 6 (1), 67-72
- **BELLON G ; MONBOISSE JC ; FANDOUX A ; BOREL JP.** *Biochim. Biophys. Acta,* 19 Août 1987, vol. 930 (1), 39-47
- **KEFALIDES NA ; CAMERON JD ; TOMICHECK EA ; YANOFF M.** *J Biol Chem.,* 10 Février 1976, vol. 251 (3), 730-3
- Ultra-structure of the human cornea. *Microscopy Research and Technique,* 1996, vol. 33, 320-335
- A new in vitro human epithelium model for assessing the eye irritation potential of formulated cosmetic products. *In vitro and Molecular Toxicology,* 1998, vol. 11 (4), 273-283